# EUROPEAN PATENT APPLICATION

(11) **EP 4 817 643 A1**
(43) Date of publication of application: **07.10.2026**
(21) Application number: 25167597.1
(22) Date of filing: 31.03.2025
(51) Int. Cl.: A61L 27/36, A61L 27/38, A61L 27/52

(54) **VASCULARIZED SUPPORTIVE MATRIX**

(71) Applicant: NORDOVO BIOSCIENCES AS, 0585 Oslo (NO)
(72) Inventor: CARASA RUIZ, Pablo., 41003 Seville (ES); TIDWELL, Tia., 4048 Hafrsfjord (NO); MORETTI, Daniele., 0576 Oslo (NO); TRAN, Anette., 8048 Zurich (CH); GAZMENO HÅTI, Armend., N-0585 Oslo (NO); SCHWEIKELE, Manuel. Boris., 0658 Oslo (NO); RAMBØL, Mia. Hauge., N-0585 Oslo (NO)
(74) Representative: Acapo Onsagers AS

(57) **Abstract**

The present invention relates to the field of regenerative medicine, in particular tissue engineering. Provided herein are improved manufacturing methodologies for incorporating functional vascular networks within complex three-dimensional matrices. The vascularized matrices are essentially non-immunogenic when implanted into a subject, mimic native extracellular matrix, facilitate suitable vascular supply throughout the matrix and may be repopulated and modulated by cells.

## Description

### Field of the invention

The present invention relates to the field of regenerative medicine, in particular tissue engineering. Provided herein are improved manufacturing methodologies for incorporating functional vascular networks within complex three-dimensional matrices. The vascularized matrices are essentially non-immunogenic when implanted into a subject, mimic native extracellular matrix, facilitate suitable vascular supply throughout the matrix and may be repopulated and modulated by cells.

### Background of the invention

Transplantation from human donors is currently the primary source for replacing damaged or dysfunctional organs and tissues in the clinic. Although transplantation has become a successful practice worldwide, the approach holds significant limitations by severe shortage of donors, complexity of the procedure and potential risk for transmission of infectious agents. As such, the use of animal tissues or synthetic materials to produce grafts has emerged as possible alternatives to human tissues. However, the disadvantages of such procedures are many and among the most important are the immunological barriers. Hence, there is a need for innovative approaches in regenerative medicine to increase the number of patients that may receive clinical implantations and with a higher rate of success.

Tissue engineering is a continuously growing multidisciplinary field applying the principles of engineering and life science to produce functional replacement tissues for clinical use by culturing cells on a supportive matrix. The field seeks to develop biological substitutes to restore, maintain or improve the biological function of a tissue or whole organ. Moreover, such tissue engineered constructs may also serve as in-vivo systems for delivery of cell-secreted molecules or treatment agents, or as in vitro models of tissue function to test various treatment effects.

The supportive matrix, also referred to as tissue scaffold in literature, that serves as an intermediate in the production of new functional tissues may present a risk of immunologic and/or inflammatory reactions when implanted. Hence, with the aim of producing more biocompatible scaffolds, techniques have been developed for using human extracellular matrix as supportive matrices for tissue culture (WO2023/187018). Extracellular matrix proteins from humans are expected to be essentially non-immunogenic when implanted into a human, mimic natural extracellular matrix compositions to achieve necessary functional tissue properties and may be repopulated and modulated by host cells. This makes human extracellular matrix proteins preferable to proteins derived from other animal sources and to synthetic degradable or non-degradable polymers.

Tissue-engineered products commercially available today have typically been limited to thin avascular tissues, such as skin and cartilage. The fabrication of thicker, more complex tissue still eludes scientists today. One reason for this is the lack of effective techniques to incorporate functional vascular networks within thick tissue constructs. Vascular networks provide cells throughout the tissue with adequate oxygen and nutrients. Cells located within thick un-vascularized tissue implants are in the risk of dying due to oxygen and nutrient deficiency. Hence, vascularization has been identified as one of the key components in the field of tissue engineering. To fabricate biomimetic tissue which accurately recapitulates our native tissue environment, *in vitro* pre-vascularization strategies need to be developed.

### Summary of the invention

The above needs have been solved by providing improved manufacturing methodologies for incorporating functional vascular networks within complex three-dimensional matrices. The vascularized matrices are essentially non-immunogenic when implanted into a subject, mimic native extracellular matrix, facilitate suitable vascular supply and may be repopulated and modulated by cells.

A **first aspect** relates to an *in-vitro* method of manufacturing vascularized extracellular matrix, the method comprising the following steps:
a) bringing a first population of cells and a second population of cells together with biocompatible hydrogel particles to obtain a cell-particle mixture;
b) culturing the cell-particle mixture to obtain the vascularized extracellular matrix;
wherein
- the first population of cells produce and secrete extracellular matrix;
- the vascularized extracellular matrix comprises capillaries embedded within the extracellular matrix;
- the second population of cells are endothelial cells that form the capillaries embedded within the extracellular matrix; and
- the number of cells in the first population of cells : number of cells in the second population of cells in step a) is in the range 1 : 10 to 10 : 1, more preferably in the range 1 : 5 to 5 : 1 and most preferably in the range 1 : 3 to 3 : 1.

### Cells

In one embodiment, the number of cells in the first population of cells : number of cells in the second population of cells in step a) may be in the range 1 : 7 to 7 : 1. More preferably, the number of cells in the first population of cells : number of cells in the second population of cells in step a) may be in the range 1 : 5 to 5 : 1. Even more preferably the number of cells in the first population of cells : number of cells in the second population of cells in step a) may be in the range 1 : 3 to 3 : 1.

In another embodiment, the number of cells in the first population of cells : number of cells in the second population of cells in step a) may be in the range 10:1 to 1:1. More preferably, the number of cells in the first population of cells : number of cells in the second population of cells in step a) may be in the range 7:1 to 1:1. Even more preferably, the number of cells in the first population of cells : number of cells in the second population of cells in step a) may be in the range 5: 1 to 1:1. Most preferably, the number of cells in the first population of cells : number of cells in the second population of cells in step a) may be in the range 3:1 to 1:1, such as about 3:1.

The cells of the first population of cells can produce and secrete extracellular matrix. It is preferred that the first population of cells are human cells. The first population of cells may be selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells (MSCs) and in particular adipose tissue derived mesenchymal stem cells or any combination thereof.

The cells in the first population of cells may be anchorage dependent cells. The biocompatible hydrogel particles may support the attachment of the first population of cells.

The second population of cells are endothelial cells that can form capillaries embedded within extracellular matrix. It is preferred that the second population of cells are human cells. The second population of cells may be cells derived from the endothelium of veins, such as cells derived from the endothelium of veins from the umbilical cord. In the most preferred embodiment, the second population of cells may be human umbilical vein endothelial cells (HUVECs).

The cells in the second population of cells may be anchorage dependent cells and the biocompatible hydrogel particles may support attachment of the second population of cells.

The cells of the first- and second populations of cells may be anchorage dependent cells and the biocompatible hydrogel particles may support attachment of the first- and/or second populations of cells.

The first population of cells may comprise MSCs, and the second population of cells may comprise HUVECs. In a preferred embodiment, the cells of the first population of cells may be MSCs and the cells of the second population of cells may be HUVECs.

### Biocompatible hydrogel particles

In one embodiment, the biocompatible hydrogel particles are biocompatible hydrogel microparticles, preferably biocompatible hydrogel microparticles of an average size less than 500 µm in their largest dimension; more preferably of an average size in the range 10 to 200 µm, such as 10 to 150 µm or 30 to 150 µm, in their largest dimension; even more preferably of an average size in the range 30 to 90 µm, such as 30 to 80 µm, in their largest dimension; and most preferably of an average size in the range 50 to 80 µm in their largest dimension. The size of the particles is measured in their swollen equilibrium state in a liquid environment of physiological ion concentration, such as cell culture medium; see example 1.2.

In one embodiment, at least 60 %, such as at least 65 %, at least 70 %, at least 75 %, at least 80 %, at least 85 %, at least 90 %, at least 95 % or at least 99 %, of the biocompatible hydrogel particles have a size in the range 10 to 200 µm in their largest dimension, such as a size in the range 10 to 150 µm in their largest dimension, a size in the range 30 to 150 µm in their largest dimension or a size in the range 30 to 90 µm in their largest dimension. The person skilled in the art will understand that if there are 100 000 biocompatible hydrogel particles, and 60 % of the biocompatible hydrogel particles have a size in the range 10 to 200 µm in their largest dimension, there are in fact 60 000 biocompatible hydrogel particles which have a size in the range 10 to 200 µm in their largest dimension.

In one embodiment, the biocompatible hydrogel particles may be self-degradable and/or self-dissolvable. In another embodiment, the method according to the first aspect further comprises a step where the vascularized extracellular matrix is subjected to treatment for dissolving and/or degrading the biocompatible hydrogel particles.

In one embodiment, the biocompatible hydrogel particles may support attachment of cells, and in particular attachment of the first population of cells and/or attachment of the second population of cells. The biocompatible hydrogel particles may have one or more cell-adhesion ligand(s) on their outer surface. The one or more cell-adhesion ligand(s) may e.g. be i) a peptide comprising a cell adhesive amino acid sequence, such as RGD, YIGSR, GFOGER or PHSRN; or ii) a charged polymer, such as poly-L-Lys. The one or more cell-adhesion ligand(s) preferably being incorporated into the biocompatible hydrogel particles via covalent tethering, electrostatic interactions, and/or interpenetrating networks. The biocompatible hydrogel particles may have one or more cell-adhesion ligand(s) on their outer surface, wherein the one or more cell-adhesion ligand(s) is a peptide comprising the amino acid sequence RGD, such as a peptide comprising the amino acid sequence GRGDSP.

The biocompatible hydrogel particles may comprise alginate, fibrin, gelatin, agarose, modified or unmodified polyethylene glycol, carrageenan, pectin or any combination thereof. It is preferred that the biocompatible hydrogel particles comprise alginate.

In another embodiment, the biocompatible hydrogel particles may comprise alginate particles, the alginate particles preferably being produced by competitive ligand exchange crosslinking e.g. as described in example 3 of WO2017153947.

In yet another embodiment, the biocompatible hydrogel particles may be alginate particles, the alginate particles preferably being produced by competitive ligand exchange crosslinking e.g. as described in example 3 of WO2017153947.

In one embodiment, the method according to the first aspect further comprises a step c), where the vascularized extracellular matrix obtained in step b) is subjected to treatment suitable for dissolving and/or degrading the biocompatible hydrogel particles.

In one embodiment, the biocompatible hydrogel particles may be alginate particles; and/or comprise alginate particles; and the method according to the first aspect further comprises a step c), where the vascularized extracellular matrix obtained in step b) is subjected to treatment suitable for dissolving and/or degrading the alginate particles. Methods suitable for dissolving and/or degrading the alginate particles are well known to the skilled person.

In one embodiment, the biocompatible hydrogel particles may be alginate particles; and/or comprise alginate particles; and the alginate particles have one or more cell-adhesion ligand(s) on their outer surface. In a preferred embodiment, said alginate is RGD-coupled alginate or GRGDSP-coupled alginate.

The biocompatible hydrogel particles may be biocompatible hydrogel beads, biocompatible hydrogel fibers or any mixture thereof. Preferably, the biocompatible hydrogel particles are biocompatible hydrogel beads.

The biocompatible hydrogel particles may be biocompatible alginate beads, biocompatible alginate fibers or any mixture thereof. Preferably, the biocompatible hydrogel particles are biocompatible alginate beads.

### Cultivation

In a preferred embodiment according to the first aspect, the cell-particle mixture in step b) is cultured in a cell media at a temperature in the range 25 to 45 °C for a predetermined period of time; and the cell media is partly or fully replaced by fresh cell media at intervals which ensures that the level of glucose in the cell media is above 1 mmol/l during the predetermined period of time, more preferably above 1.1 mmol/l during the predetermined period of time and even more preferably above 1.2 mmol/l during the predetermined period of time. The predetermined period of time may be more than 7 days, such as more than 15 days.

In one embodiment, the cell-particle mixture in step b) of the method according to the first aspect is cultured in a cell media at a temperature in the range 25 to 45 °C for a period of time less than 35 days, more preferably less than 30 days, even more preferably less than 25 days and most preferably less than 21 days, such as less than 18 days or less than 14 days.

### Mold

In a preferred embodiment according to the first aspect, the cell-particle mixture in step b) is cultured in a mold. The mold is preferably made of a bioinert material.

In one embodiment, the mold has one or more of the following properties:
- allow control of geometry;
- prevent cell attachment and/or cell infiltration; and
- allow the exchange of nutrients and gases between exterior and interior of the mold.

The mold may be designed to prevent cell attachment and/or cell infiltration. Further, the mold may allow the exchange of nutrients and/or gases between exterior and interior of the mold. Preferably, the mold is completely or partly submerged in cell culture media during use.

In a preferred embodiment, the mold is designed to prevent cell attachment and/or cell infiltration; allows exchange of nutrients and gases between exterior and interior of the mold; and is completely or partly submerged in cell culture media. The mold may have the shape of an annular well.

In a preferred embodiment, the mold is the cell culture apparatus disclosed in EP24214592, which is hereby incorporated by reference in its entirety. The cell culture apparatus, which is depicted in figure 5 herein (figure 5 herein corresponds to figure 3 in EP24214592), comprises a cell media reservoir (36) and a tissue cassette (2), wherein the cell media reservoir comprises an enclosure for enclosing cell culture media, wherein the cell media reservoir has an opening for inserting the tissue cassette into the cell media enclosed in the cell media reservoir, wherein the tissue cassette has a cassette housing comprising a first main side having a first aperture covered by a first membrane, and at least one second main side having a second aperture covered by a second membrane, such that a culture chamber is formed between the first membrane and the second membrane, wherein the first membrane and the second membrane are attached to the cassette housing, wherein the tissue cassette has an injection opening extending through the cassette housing into the culture chamber for feeding the cell-particle mixture into the culture chamber, and wherein at least one of the first membrane and the second membrane is gas-permeable.

A **second aspect** relates to a vascularized extracellular matrix obtainable or obtained by the method according to the first aspect or any of the above-mentioned embodiments.

The vascularized extracellular matrix obtainable or obtained by the method according to the first aspect may comprise extracellular matrix, preferably human extracellular matrix, and capillaries embedded within the extracellular matrix.

The vascularized extracellular matrix obtainable or obtained by the method according to the first aspect may comprise:
- extracellular matrix, preferably human extracellular matrix;
- capillaries embedded within the extracellular matrix;
- a first population of cells embedded within the extracellular matrix;
- a second population of cells embedded within the extracellular matrix; and
- biocompatible hydrogel particles embedded within the extracellular matrix;
wherein
- the first population of cells produce and secrete the extracellular matrix; and
- the second population of cells are endothelial cells that form the capillaries embedded within the extracellular matrix.

A **third aspect** relates to a vascularized extracellular matrix comprising:
- extracellular matrix, preferably human extracellular matrix;
- capillaries embedded within the extracellular matrix;
- a first population of cells embedded within the extracellular matrix;
- a second population of cells embedded within the extracellular matrix; and
- biocompatible hydrogel particles embedded within the extracellular matrix; wherein
- the first population of cells produce and secrete the extracellular matrix; and
- the second population of cells are endothelial cells that form the capillaries embedded within the extracellular matrix.

### Cells

The cells of the first population of cells can produce and secrete extracellular matrix. It is preferred that the first population of cells are human cells. The first population of cells may be selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells (MSCs) and in particular adipose tissue derived mesenchymal stem cells or any combination thereof.

The cells in the first population of cells may be anchorage dependent cells, the vascularized extracellular matrix may further comprise biocompatible hydrogel particles, and the biocompatible hydrogel particles may support attachment of the first population of cells. In one embodiment, at least one of the cells in the first population of cells is attached to the biocompatible hydrogel particles.

The second population of cells are endothelial cells that can form capillaries embedded within extracellular matrix. It is preferred that the second population of cells are human cells. The second population of cells may be cells derived from the endothelium of veins, such as cells derived from the endothelium of veins from the umbilical cord. In the most preferred embodiment, the second population of cells are Human Umbilical Vein Endothelial Cells (HUVECs).

The cells in the second population of cells may be anchorage dependent cells, the vascularized extracellular matrix may further comprise biocompatible hydrogel particles, and the biocompatible hydrogel particles may support attachment of the second population of cells. In one embodiment, at least one of the cells in the second population of cells is attached to the biocompatible hydrogel particles.

The cells in the first- and second populations of cells may be anchorage dependent cells, the vascularized extracellular matrix may further comprise biocompatible hydrogel particles, and the biocompatible hydrogel particles may support attachment of the first- and/or second populations of cells. In one embodiment, the vascularized extracellular matrix further comprises biocompatible hydrogel particles; and at least one of the cells in the first population of cells and/or at least one of the cells in the second population of cells is attached to the biocompatible hydrogel particles.

The first population of cells may comprise MSCs and the second population of cells may comprise HUVECs. In a preferred embodiment, the cells of the first population of cells are MSCs and the cells of the second population of cells are HUVECs.

### Biocompatible hydrogel particles

In one embodiment, the vascularized extracellular matrix according to the third aspect further comprises biocompatible hydrogel particles.

In one embodiment, the biocompatible hydrogel particles are biocompatible hydrogel microparticles, preferably biocompatible hydrogel microparticles of an average size less than 500 µm in their largest dimension; more preferably of an average size in the range 10 to 200 µm, such as 10 to 150 µm or 30 to 150 µm, in their largest dimension; even more preferably of an average size in the range 30 to 90 µm, such as 30 to 80 µm, in their largest dimension; and most preferably of an average size in the range 50 to 80 µm in their largest dimension. The size of the particles is measured in their swollen equilibrium state in a liquid environment of physiological ion concentration, such as cell culture medium; see example 1.2.

In one embodiment, at least 60 %, such as at least 65 %, at least 70 %, at least 75 %, at least 80 %, at least 85 %, at least 90 %, at least 95 % or at least 99 %, of the biocompatible hydrogel particles have a size in the range 10 to 200 µm in their largest dimension, such as a size in the range 10 to 150 µm in their largest dimension, a size in the range 30 to 150 µm in their largest dimension or a size in the range 30 to 90 µm in their largest dimension. The person skilled in the art will understand that if there are 100 000 biocompatible hydrogel particles, and 60 % of the biocompatible hydrogel particles have a size in the range 10 to 200 µm in their largest dimension, there are in fact 60 000 biocompatible hydrogel particles which have a size in the range 10 to 200 µm in their largest dimension.

In one embodiment, the biocompatible hydrogel particles may be self-degradable and/or self-dissolvable. In another embodiment, the method according to the first aspect further comprises a step where the vascularized extracellular matrix is subjected to treatment for dissolving and/or degrading the biocompatible hydrogel particles.

In one embodiment, the biocompatible hydrogel particles may support attachment of cells, and in particular attachment of the first population of cells and/or attachment of the second population of cells. The biocompatible hydrogel particles may have one or more cell-adhesion ligand(s) on their outer surface. The one or more cell-adhesion ligand(s) may e.g. be i) a peptide comprising a cell adhesive amino acid sequence, such as RGD, YIGSR, GFOGER or PHSRN; or ii) a charged polymer, such as poly-L-Lys. The one or more cell-adhesion ligand(s) preferably being incorporated into the biocompatible hydrogel particles via covalent tethering, electrostatic interactions, and/or interpenetrating networks. The biocompatible hydrogel particles may have one or more cell-adhesion ligand(s) on their outer surface, wherein the one or more cell-adhesion ligand(s) is a peptide comprising the amino acid sequence RGD, such as a peptide comprising the amino acid sequence GRGDSP.

The biocompatible hydrogel particles may comprise alginate, fibrin, gelatin, agarose, modified or unmodified polyethylene glycol, carrageenan, pectin or any combination thereof. It is preferred that the biocompatible hydrogel particles comprise alginate.

In another embodiment, the biocompatible hydrogel particles may comprise alginate particles, the alginate particles preferably being produced by competitive ligand exchange crosslinking e.g. as described in example 3 of WO2017153947.

In yet another embodiment, the biocompatible hydrogel particles may be alginate particles, the alginate particles preferably being produced by competitive ligand exchange crosslinking e.g. as described in example 3 of WO2017153947.

In one embodiment, the method according to the first aspect further comprises a step c), where the vascularized extracellular matrix obtained in step b) is subjected to treatment suitable for dissolving and/or degrading the biocompatible hydrogel particles.

In one embodiment, the biocompatible hydrogel particles may be alginate particles; and/or comprise alginate particles; and the method according to the first aspect further comprises a step c), where the vascularized extracellular matrix obtained in step b) is subjected to treatment suitable for dissolving and/or degrading the alginate particles. Methods suitable for dissolving and/or degrading the alginate particles are well known to the skilled person.

In one embodiment, the biocompatible hydrogel particles may be alginate particles; and/or comprise alginate particles; and the alginate particles have one or more cell-adhesion ligand(s) on their outer surface. In a preferred embodiment, said alginate is RGD-coupled alginate or GRGDSP-coupled alginate.

The biocompatible hydrogel particles may be biocompatible hydrogel beads, biocompatible hydrogel fibers or any mixture thereof. Preferably, the biocompatible hydrogel particles are biocompatible hydrogel beads.

The biocompatible hydrogel particles may be biocompatible alginate beads, biocompatible alginate fibers or any mixture thereof. Preferably, the biocompatible hydrogel particles are biocompatible alginate beads.

A **fourth aspect** relates to a composition comprising:
- a first population of cells;
- a second population of cells; and
- biocompatible hydrogel particles;
wherein
- the first population of cells can produce and secrete extracellular matrix;
- the second population of cells being endothelial cells that can form capillaries within extracellular matrix; and
- the number of cells in the first population of cells : number of cells in the second population of cells is in the range 1 : 10 to 10 : 1, more preferably in the range 1 : 5 to 5 : 1 and most preferably in the range 1 : 3 to 3 : 1.

### Cells

In one embodiment, the number of cells in the first population of cells : number of cells in the second population of cells is in the range 1 : 7 to 7 : 1. More preferably, the number of cells in the first population of cells : number of cells in the second population of cells is in the range 1 : 5 to 5 : 1. Even more preferably the number of cells in the first population of cells : number of cells in the second population of cells is in the range 1 : 3 to 3 : 1.

The number of cells in the first population of cells : number of cells in the second population of cells may be in the range 10:1 to 1:1. The number of cells in the first population of cells : number of cells in the second population of cells may be in the range 7:1 to 1:1. The number of cells in the first population of cells : number of cells in the second population of cells may be in the range 5:1 to 1:1. Most preferably, the number of cells in the first population of cells : number of cells in the second population of cells is in the range 3: 1 to 1:1, such as a ratio about 3:1.

The cells of the first population of cells can produce and secrete extracellular matrix. It is preferred that the first population of cells are human cells. The first population of cells may be selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells (MSCs) and in particular adipose tissue derived mesenchymal stem cells or any combination thereof.

The cells in the first population of cells may be anchorage dependent cells, and the biocompatible hydrogel particles may support attachment of the first population of cells. In one embodiment, at least one of the cells in the first population of cells is attached to the biocompatible hydrogel particles.

The second population of cells may be endothelial cells that can form capillaries within extracellular matrix. It is preferred that the second population of cells are human cells. The second population of cells may be cells derived from the endothelium of veins, such as cells derived from the endothelium of veins from the umbilical cord. In the most preferred embodiment, the second population of cells may be human umbilical vein endothelial cells (HUVECs).

The cells in the second population of cells may be anchorage dependent cells, and the biocompatible hydrogel particles may support attachment of the second population of cells. In one embodiment, at least one of the cells in the second population of cells is attached to the biocompatible hydrogel particles.

The cells in the first- and second populations of cells may be anchorage dependent cells, and the biocompatible hydrogel particles may support attachment of the first- and/or second populations of cells. In one embodiment, at least one of the cells in the first population of cells and/or at least one of the cells in the second population of cells is attached to the biocompatible hydrogel particles.

The first population of cells may comprise MSCs and the second population of cells may comprise HUVECs. In a preferred embodiment, the cells of the first population of cells are MSCs and the cells of the second population of cells are HUVECs.

### Biocompatible hydrogel particles

In one embodiment, the biocompatible hydrogel particles are biocompatible hydrogel microparticles, preferably biocompatible hydrogel microparticles of an average size less than 500 µm in their largest dimension; more preferably of an average size in the range 10 to 200 µm, such as 10 to 150 µm or 30 to 150 µm, in their largest dimension; even more preferably of an average size in the range 30 to 90 µm, such as 30 to 80 µm, in their largest dimension; and most preferably of an average size in the range 50 to 80 µm in their largest dimension. The size of the particles is measured in their swollen equilibrium state in a liquid environment of physiological ion concentration, such as cell culture medium; see example 1.2.

In one embodiment, at least 60 %, such as at least 65 %, at least 70 %, at least 75 %, at least 80 %, at least 85 %, at least 90 %, at least 95 % or at least 99 %, of the biocompatible hydrogel particles have a size in the range 10 to 200 µm in their largest dimension, such as a size in the range 10 to 150 µm in their largest dimension, a size in the range 30 to 150 µm in their largest dimension or a size in the range 30 to 90 µm in their largest dimension. The person skilled in the art will understand that if there are 100 000 biocompatible hydrogel particles, and 60 % of the biocompatible hydrogel particles have a size in the range 10 to 200 µm in their largest dimension, there are in fact 60 000 biocompatible hydrogel particles which have a size in the range 10 to 200 µm in their largest dimension.

In one embodiment, the biocompatible hydrogel particles may support attachment of cells, and in particular attachment of the first population of cells and/or attachment of the second population of cells. The biocompatible hydrogel particles may have one or more cell-adhesion ligand(s) on their outer surface. The one or more cell-adhesion ligand(s) may e.g. be i) a peptide comprising a cell adhesive amino acid sequence, such as RGD, YIGSR, GFOGER or PHSRN; or ii) a charged polymer, such as poly-L-Lys. The one or more cell-adhesion ligand(s) preferably being incorporated into the biocompatible hydrogel particles via covalent tethering, electrostatic interactions, and/or interpenetrating networks. The biocompatible hydrogel particles may have one or more cell-adhesion ligand(s) on their outer surface, wherein the one or more cell-adhesion ligand(s) is a peptide comprising the amino acid sequence RGD, such as a peptide comprising the amino acid sequence GRGDSP.

In one embodiment, the biocompatible hydrogel particles support attachment of cells, preferably by having one or more cell-adhesion ligand(s) on their outer surface; and at least one of the cells of the first population of cells and/or at least one of the cells of the second population of cells being attached to the biocompatible hydrogel particles.

In one embodiment, the biocompatible hydrogel particles may be alginate particles; and/or comprise alginate particles; and the alginate particles have one or more cell-adhesion ligand(s) on their outer surface. In a preferred embodiment, said alginate is RGD-coupled alginate or GRGDSP-coupled alginate.

In one embodiment, the biocompatible hydrogel particles may be self-degradable and/or self-dissolvable.

The biocompatible hydrogel particles may comprise alginate, fibrin, gelatin, agarose, modified or unmodified polyethylene glycol, carrageenan, pectin or any combination thereof. It is preferred that the biocompatible hydrogel particles comprise alginate.

In another embodiment, the biocompatible hydrogel particles may comprise alginate particles, the alginate particles preferably being produced by competitive ligand exchange crosslinking e.g. as described in example 3 of WO2017153947.

In yet another embodiment, the biocompatible hydrogel particles may be alginate particles, the alginate particles preferably being produced by competitive ligand exchange crosslinking e.g. as described in example 3 of WO2017153947.

The biocompatible hydrogel particles may be biocompatible hydrogel beads, biocompatible hydrogel fibers or any mixture thereof. Preferably, the biocompatible hydrogel particles are biocompatible hydrogel beads.

The biocompatible hydrogel particles may be biocompatible alginate beads, biocompatible alginate fibers or any mixture thereof. Preferably, the biocompatible hydrogel particles are biocompatible alginate beads.

A **fifth aspect** relates to *in-vitro* use of the vascularized extracellular matrix according to the second or third aspect for growing bioartificial tissue, in particular as a supportive matrix for growing bioartificial tissue.

A **sixth aspect** relates to the vascularized extracellular matrix according to the second or third aspect for use in treatment of skin wounds.

A **seventh aspect** relates to the vascularized extracellular matrix according to the second or third aspect for use in a surgical procedure for replacing and/or repairing blood vessels in a subject, preferably a human subject.

### Definitions

The terms "self-degradable" and "self- dissolvable" means that the biocompatible hydrogel particle will degrade or dissolve by itself. Thus, there is no need for adding any agents for the degradation or dissolution to take place. The time needed for the degradation or dissolution to be completed will typically be decreased at increasing temperature.

The expression "anchorage dependent cells" as used herein are any type of cells that will grow, survive, maintain function or differentiate when attached to a surface, also known as substrate-dependent cells.

The term "hydrogel" as used herein refers to a polymer network that can absorb and retain large amounts of water while maintaining structural integrity. The hydrogel may be synthetic or derived from nature.

The terms "extracellular matrix" and "ECM" are used interchangeably herein to describe the intricate network composed of an array of multidomain macromolecules, such as collagen, elastin, enzymes, glycoproteins and hydroxyapatite, organized in a cell/tissue-specific manner.

The terms "bioinert" or "inert" as used herein is interchangeably used to describe the feature of being deficient in active properties, i.e. lacking a usual or anticipated chemical or biological action.

The term "microparticle" as used herein refers to a particle less than 1000 µm in its largest dimension.

The term "hydrogel particle" includes both hydrogel bead and hydrogel fiber.

### Abbreviations

- MSC: Mesenchymal Stem Cells
- HUVEC: Human Umbilical Vein Endothelial Cells
- RCF: In centrifugation, the term RCF (Relative Centrifugal Force) denotes the degree of force exerted on a sample. RCF is quantified in multiples of the gravitational acceleration that is standard at the Earth's surface (x g).
- NBF: Neutral-buffered formalin
- ECM: Extracellular matrix

### Brief description of the drawings

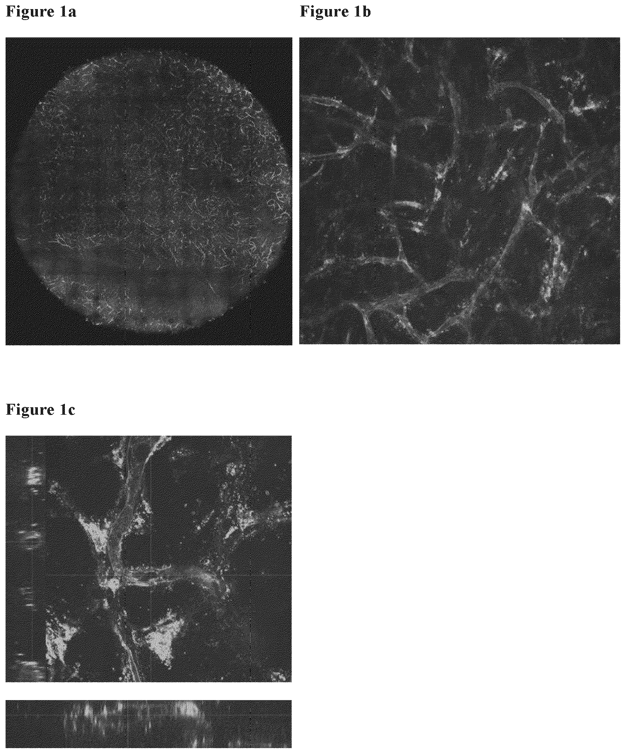

**Figures 1a, 1b and 1c** are representative confocal images of a fraction of the vascularized extracellular matrix prepared according to example 1.3 and stained with ULEX-AF488 and Hoechst 33420. Figure 1a clearly shows that capillaries have formed throughout the vascularized extracellular matrix (10X magnification). From figure 1b it is apparent that the capillaries have distinct walls (20X magnification). Figure 1c shows the lumen of one capillary (40X magnification; orthogonal projection in the lower figure).

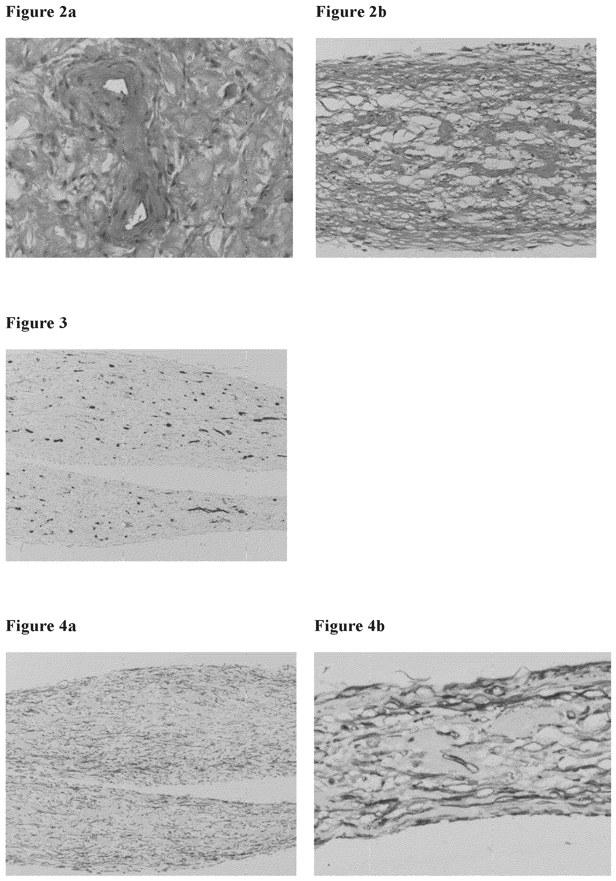

**Figures 2a and 2b** show the results of a histological analysis (staining by Hematoxylin & Eosin) of the vascularized extracellular matrix prepared according to example 1.3. General organization of the vascularized extracellular matrices is clearly visible, with MSCs forming collagen fibres mostly on the outer layers and having a looser inner structure. Figure 2a represents a longitudinal section with 20X magnification, where it is possible to observe structures resembling capillaries. Figure 2b represents cross section with 20X magnification. Reference is made to the protocol disclosed in example 1.4.
**Figure 3** shows immunohistochemical (IHC) staining for CD31 of the vascularized extracellular matrix prepared according to example 1.3. CD31 is expressed as expected and there are clear indications of capillary-like structures (10X magnification). Reference is made to the protocol disclosed in example 1.4.
**Figure 4a and 4b** show immunohistochemical (IHC) staining for Smooth Muscle Actin (SMA) of the vascularized extracellular matrix prepared according to example 1.3. Most of the MSCs seems to express this marker, indicating their initial transition to myofibroblasts (doi.org/10.1016/j.stemcr.2015.05.004). Reference is made to the protocol disclosed in example 1.4.

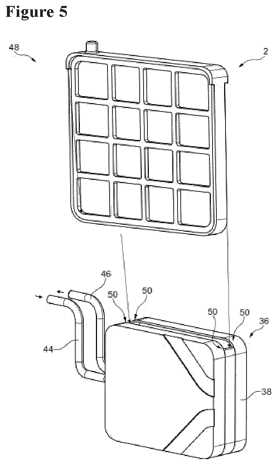

**Figure 5** shows a cell culture apparatus (48), which substantially consists of a cell media reservoir (36) and a tissue cassette (2). The width w of the tissue cassette (2) and the associated dimension of the insertion opening (14) are adapted to each other, such that the tissue cassette (2) can be easily inserted into the insertion opening (40). In doing so, the shoulders (34) rest on edge regions (50) of the cell media reservoir (38) and the tissue cassette (2) are held in the cell media reservoir (36). Further details about the cell culture apparatus (48) are disclosed in EP24214592.

### Detailed description of the invention

Unless specifically defined herein, all technical and scientific terms used have the same meaning as commonly understood by a skilled artisan in the fields of regenerative medicine, tissue engineering, biochemistry, and molecular biology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail.

Where a numerical limit or range is stated herein, the endpoints are included. Also, all values and sub ranges within a numerical limit or range are specifically included as if explicitly written out.

Tissue engineering as a field seeks to develop biological substitutes to restore the biological function of a tissue or whole organ and involves methods of culturing cells on a supportive matrix to form new tissues of intended shape and function. However, there are several shortcomings to the method, and clinical translation of such engineered tissues is low.

The supportive matrix that serves as an intermediate in the production of a new functional tissue may present a risk of immunologic and/or inflammatory reactions when implanted. Hence, it is important that the supportive matrix is essentially non-immunogenic when implanted into a subject. The supportive matrix should also facilitate suitable vascular supply throughout the supportive matrix, and it represents an advantage if the supportive matrix mimics native extracellular matrix. The supportive matrix should also be capable of being repopulated and modulated by cells. It is also desirable that a supportive matrix of any geometrical shape may be manufactured. Consequently, there is a need in the art to develop new methodologies for manufacturing vascularized supportive matrices for use in tissue engineering.

The present inventors have solved the above needs by providing improved manufacturing methodologies for incorporating functional vascular networks within complex three-dimensional matrices. These supportive matrices are essentially non-immunogenic when implanted into a human, mimic native extracellular matrix, facilitate suitable vascular supply throughout the matrix, and may be repopulated and modulated by cells.

In general, the present invention involves manufacturing of a vascularized extracellular matrix which may find use as a supportive matrix in tissue engineering. The vascularized extracellular matrix has been developed primarily by *in vitro* culturing of a first- and second population of cells in the presence of biocompatible hydrogel particles. The first population of cells produce and secrete extracellular matrix, the second population of cells are endothelial cells that form capillaries embedded within the extracellular matrix, and the space between the biocompatible hydrogel particles creates a porous structure which allows for diffusion of waste and nutrients throughout the vascularized extracellular matrix. The biocompatible hydrogel particles may also adhere to the cells thereby facilitating cell-to-cell contact. Further, the biocompatible hydrogel particles may easily be removed from the vascularized extracellular matrix in case the intended use requires a product without hydrogel particles. The vascularized extracellular matrix may be repopulated and modulated by cells, either *in-vitro* or by host cells *in-vivo.*

Thus, **a first aspect** provides an *in-vitro* method of manufacturing vascularized extracellular matrix, the method comprising the following steps:
a) bringing a first population of cells and a second population of cells together with biocompatible hydrogel particles to obtain a cell-particle mixture;
b) culturing the cell-particle mixture to obtain the vascularized extracellular matrix;
wherein
- the first population of cells produce and secrete extracellular matrix;
- the vascularized extracellular matrix comprises capillaries embedded within the extracellular matrix;
- the second population of cells being endothelial cells that form the capillaries embedded within the extracellular matrix; and
- the number of cells in the first population of cells : number of cells in the second population of cells, in step a), is in the range 1 : 10 to 10 : 1, more preferably in the range 10 : 1.

The first step of the method refers to a first- and a second population of cells, and biocompatible hydrogel particles. The biocompatible hydrogel particles may support cell attachment, and one or more cells of the first population of cells and/or one or more cells of the second population of cells may adhere to the biocompatible hydrogel particles. The first population of cells produce and secrete extracellular matrix, which is the primary building block of the vascularized extracellular matrix.

The second population of cells are endothelial cells that form the capillaries embedded within the extracellular matrix.

**The first population of cells** may be derived from an animal or human donor or from an established cell line. Preferably, the cells are of the same species as the intended recipient, e.g. to reduce immunologic reactions and increase compatibility of the supportive matrix with recipient. Rigorous screening of the cells for transmissible diseases (e.g., HIV or hepatitis), further decrease the infectious risk associated with the supportive matrix products. The cells may further have desirable properties such as an ability to grow well in culture, or the cells are genetically modified to improve their secretion of extracellular matrix components.

The first population of cells may in principle be any type of cells, preferably anchorage-dependent cells, that will grow, survive, and secrete extracellular matrix. It is preferred that the cells of the first population of cells produce and secrete as much extracellular matrix as possible. Hence, the first population of cells is preferably selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells (MSCs) and in particular adipose tissue derived mesenchymal stem cells, or any combination thereof. In a preferred embodiment, the first group of cells is adipose tissue derived mesenchymal stem cells. In one embodiment the first group of cells have been genetically modified to increase their secretion of extracellular matrix.

**The second population of cells** may be derived from an animal or human donor or from an established cell line. Preferably, the cells are of the same species as the intended recipient, e.g. to reduce immunologic reactions and increase compatibility of the supportive matrix with recipient. Rigorous screening of the cells for transmissible diseases (e.g., HIV or hepatitis), further decrease the infectious risk associated with the supportive matrix products. The cells may further have desirable properties such as an ability to grow well in culture, or the cells are genetically modified for improved vascularization within extracellular matrix produced and secreted by the first population of cells.

The second population of cells are endothelial cells that can form capillaries within the extracellular matrix. The cells may be derived from the endothelium of veins, such as cells derived from the endothelium of veins from the umbilical cord. In the most preferred embodiment, the second population of cells may be human umbilical vein endothelial cells (HUVECs).

The first- and second population of cells may be of different or the same species. However, it is preferred that both the first- and second population of cells are of the same species as the intended recipient, e.g. to reduce risk of immunologic reactions and increase compatibility of the vascularized extracellular matrix with recipient.

**The ratio** between 'the number of cells in the first population of cells' and 'the number of cells in the second population of cells' in step a) may have an effect on the yield and the quality of the product. Extracellular matrix is the primary building block of the vascularized extracellular matrix, and it therefore seems reasonable to assume that the number of cells in the first population of cells should be high in order to have an effective production of extracellular matrix. However, the first population of cells typically proliferate fast in culture while growth of the second population of cells is more limited due to the need for self-organizing into capillaries. An outgrowth of the first population of cells may limit nutrient access to the second population of cells over time, which may lead the second population of cells to a premature death. Hence, even if there is a broad range of ratios that are acceptable, it may represent an advantage to have an appropriate balance between 'the number of cells in the first population of cells' and 'the number of cells in the second population of cells'.

Example 1.3 demonstrates effective manufacturing of a vascularized extracellular matrix by bringing 75 × 10⁶ MSCs and 25 x 10⁶ HUVECs together with alginate microbeads. A ratio of 3 : 1 between the number of cells in the first population of cells (75 x 10⁶ MSCs) and the number of cells in the second population of cells (25 x 10⁶ HUVECs) represents a preferred embodiment. In view of that result, it should be expected that also a ratio of 10 : 1 (number of cells in the first population of cells : number of cells in the second population of cells) would be acceptable, even though the second population of cells may to some extent experience reduced levels of nutrients over time. Since the cells of the first population of cells typically proliferate faster in culture compared to cells of the second population of cells, it seems reasonable to assume that a ratio of 1 : 10 between the number of cells in the first population of cells and the number of cells in the second population of cells is acceptable, even though less efficient production of extracellular matrix should be expected. Hence, the number of cells in the first population of cells : number of cells in the second population of cells in step a) of the first aspect may be in the range 1 : 10 to 10 : 1, preferably in the range 1 : 5 to 5 : 1 and more preferably in the range 1 : 3 to 3 : 1.

**The biocompatible hydrogel particles** may support the attachment of cells; and one or more cells of the first population of cells and/or one or more cells of the second population of cells may adhere to the biocompatible hydrogel particles.

Hence, the biocompatible hydrogel particles may be an initial substrate for the first population of cells and/or the second population of cells to grow on.

In order for the cells, in particular the first population of cells, to attach to the biocompatible hydrogel particles, the biocompatible hydrogel particles need to support cell attachment. If the biocompatible hydrogel particles do not support cell attachment, one or more cell-adhesion ligand(s) may be incorporated into the biocompatible hydrogel particles or coated on the surface of the biocompatible hydrogel particles to achieve proper conditions for attachment, herein also called functionalization of the biocompatible hydrogel particles. For example, covalently conjugated biocompatible hydrogel particles with heparin binding peptides or peptide sequences found in extracellular matrix proteins can be used to tailor the type and degree of interaction with cells. Interaction of cells with biocompatible hydrogel particles comprising cell-adhesion ligand(s) is often mediated through cellular receptors on the surface of the cells recognizing the cell adhesion molecules at the surface of the biocompatible hydrogel particles. Hence, in one embodiment the biocompatible hydrogel particles have one or more cell-adhesion ligand(s) on their outer surface.

Cells, in particular anchorage dependent cells, do not readily attach and grow on alginate hydrogel particles. However, free hydroxyl and carboxyl groups are distributed along the backbone of alginate, rendering alginate suitable for chemical modification, e.g. covalently conjugating alginate with cell-adhesion ligand(s).

According to one embodiment, the biocompatible hydrogel particles comprise alginate particles, and more preferably the biocompatible hydrogel particles are alginate particles; wherein the alginate particles comprise one or more cell-adhesion ligand(s) on their outer surface. The one or more cell-adhesion ligand(s) may e.g. be i) a peptide comprising a cell adhesive amino acid sequence, such as RGD, YIGSR, GFOGER or PHSRN; or ii) a charged polymer, such as poly-L-Lys. The one or more cell-adhesion ligand(s) preferably being incorporated into the biocompatible hydrogel particles via covalent tethering, electrostatic interactions, and/or interpenetrating networks. Peptide coupled alginates are e.g. available from Novamatrix^{®} (https://novamatrix.biz/).

Functionalization of the biocompatible hydrogel particles enables not only attachment of one cell to one particle, but various attachment of one or more cells to one or more particles, and cell-to-cell attachment. Hence, neighboring biocompatible hydrogel particles may be connected to one another by a network of cells and the extracellular matrix thereby forming a three-dimensional lattice like structure.

Hydrogels, like alginate, are optimal substrates for biomedical applications as they are crosslinked hydrophilic polymers that themselves do not dissolve in water, and are highly absorbent while still maintaining a well-defined structure. This is a beneficial characteristic when serving as a substrate to culture cells as the hydrogel itself may serve as a reservoir for nutrients deep within the supportive matrix.

Further, the spacing between the biocompatible hydrogel particles creates a porous structure which allows for diffusion of nutrients and waste throughout the supportive matrix as the cells grow and secret extracellular matrix. This provides an advantage over bulk gels used in prior art in that also supportive matrices of greater thickness can be manufactured. The extended ability for diffusion of nutrients and waste by using biocompatible hydrogel particles therefore provides the advantage of enabling production of complex three-dimensional supportive matrices of greater thickness.

The person skilled in the art will acknowledge that the size of the biocompatible hydrogel particles likely will affect i) the porosity of the structure, i.e. spacing between the biocompatible hydrogel particles; and ii) the surface area to volume ratio of the particles. While increased particle size would be expected to increase the volume of the spacing between the particles, the surface area to volume ratio of the particles would be expected to decrease. Thus, increasing the size of the particles would likely improve diffusion of nutrients and waste throughout the supportive matrix but at the same time cause less efficient production of extracellular matrix. Thus, the size of the biocompatible hydrogel particles should be selected for optimal waste and nutrient diffusion, and optimal number of cells per volume.

In one embodiment, the biocompatible hydrogel particles are biocompatible hydrogel microparticles, preferably biocompatible hydrogel microparticles of an average size less than 500 µm in their largest dimension; more preferably of an average size in the range 10 to 200 µm, such as 10 to 150 µm or 30 to 150 µm, in their largest dimension; even more preferably of an average size in the range 30 to 90 µm, such as 30 to 80 µm, in their largest dimension; and most preferably of an average size in the range 50 to 80 µm in their largest dimension. The size of the particles is measured in their swollen equilibrium state in a liquid environment of physiological ion concentration, such as cell culture medium; see example 1.2.

In one embodiment, at least 60 %, such as at least 65 %, at least 70 %, at least 75 %, at least 80 %, at least 85 %, at least 90 %, at least 95 % or at least 99 %, of the biocompatible hydrogel particles have a size in the range 10 to 200 µm in their largest dimension, such as a size in the range 10 to 150 µm in their largest dimension, a size in the range 30 to 150 µm in their largest dimension or a size in the range 30 to 90 µm in their largest dimension. The person skilled in the art will understand that if there are 100 000 biocompatible hydrogel particles, and 60 % of the biocompatible hydrogel particles have a size in the range 10 to 200 µm in their largest dimension, there are in fact 60 000 biocompatible hydrogel particles which have a size in the range 10 to 200 µm in their largest dimension.

As previously discussed, hydrogels are highly absorbent and the size of the particles is therefore measured in their swollen equilibrium state in a liquid environment of physiological ion concentration, such as cell culture medium. Example 1.2 provides a technique for measuring the diameter of hydrogel beads.

Biocompatible hydrogels may be produced by any method of cross-linking polymers, such as free-radical polymerization, chemical reaction or ionizing radiation. In the case of polyvalent, in particular divalent, ion cross-linking hydrogels, it is preferred that the biocompatible hydrogel particles are prepared by competitive ligand exchange as described in WO2017153947, in particular as described in example 1 and example 3 of said document.

The biocompatible hydrogel particles may be made from or comprise alginate; fibrin; gelatin; agarose; modified or unmodified poly-ethylene glycol; carrageenan; pectin; hyaluronic acid; collagen; cellulose derivatives, such as e.g. hydroxy methyl propyl cellulose (HPMC) or methyl propyl cellulose (MPC); or any combination thereof. The polymers used in production of biocompatible hydrogel particles may be degradable or dissolvable and may have inherently low immunogenicity. The biocompatible hydrogel particles may be prepared from natural polymers. In a preferred embodiment, the biocompatible hydrogel particles comprise alginate particles, and even more preferably the biocompatible hydrogel particles are alginate particles.

**Alginate** has been used for many biomedical applications due to its biocompatibility, low toxicity, and ease of gelation by addition of divalent cations such as e.g. Ca²⁺. Alginate hydrogels can be prepared by various cross-linking methods well known to the skilled person, including competitive ligand exchange as discussed above, from a variety of commercially available alginate, typically obtained from brown algae, such as e.g. *Laminaria hyperborean, Laminaria digitatat, Laiminaria japonica, Ascopyllum nodosum* or *Macrocystis pyrifera.*

Alginate is a naturally occurring anionic polymer containing blocks of (1,4)-linked β-D-mannuronate (M) and α-L-guluronate (G) residues. The blocks are composed of consecutive G residues (GGGGGG), consecutive M residues (MMMMMM), and alternating M and G residues (GMGMGM). Alginates extracted from different sources differ in M and G contents as well as the length of each block, and more than 200 different alginates are currently being manufactured

Only the G-blocks of alginate are believed to participate in intermolecular cross-linking with divalent cations (e.g., Ca²⁺) to form hydrogels. The composition (i.e., M/G ratio), sequence, G-block length, and molecular weight are thus factors affecting the physical properties of alginate and its resultant hydrogels.

The mechanical properties of alginate gels typically are enhanced by increasing the length of G-block and molecular weight. However, an alginate solution formed from high molecular weight polymer also becomes highly viscous. Manipulation of the molecular weight and its distribution can independently control the pre-gel solution viscosity and post-gelling stiffness. The elastic modulus of gels can be increased significantly, while the viscosity of the solution minimally raised, by using a combination of high and low molecular weight alginate polymers.

The skilled person will acknowledge that the proportions of M and G residues varies depending upon the species of brown alga from which the alginate is obtained. The skilled person will also acknowledge that some alginates having high proportions of G-residues may commonly be referred to as G-rich alginate, whereas alginates having low proportions of G-residues may commonly be referred to as M-rich alginate.

The biocompatible hydrogel particles may be made of alginate having high proportions of G-residues, i.e. G-rich alginate. According to one embodiment, the biocompatible hydrogel particles are made of alginate comprising ≥ 30% G-residues, such as ≥ 35% G-residues, ≥ 40% G-residues, ≥ 45% G-residues, ≥ 50% G-residues, ≥ 55% G-residues, ≥ 60% G-residues, ≥ 65% G-residues or ≥ 70% G-residues (monomer units). In a preferred embodiment, the biocompatible hydrogel particles are made of alginate comprising ≥ 40% G-residues, such as ≥ 45% G-residues (monomer units).

The biocompatible hydrogel particles may be made of a mixture of at least two different alginates, wherein each of the alginates independently comprises ≥ 30% G-residues, such as ≥ 35% G-residues, ≥ 40% G-residues, ≥ 45% G-residues, ≥ 50% G-residues, ≥ 55% G-residues, ≥ 60% G-residues, ≥ 65% G-residues or ≥ 70% G-residues (monomer units).

In another embodiment, the biocompatible hydrogel particles are made of alginate comprising 30-90 % G-residues, such as 30-80 % G-residues, 40-80 % G-residues or 40-75 % G-residues (monomer units).

In one embodiment, the biocompatible hydrogel particles are made of a mixture of at least two different alginates, wherein each of the alginates independently comprises 30-90 % G-residues, such as 30-80 % G-residues, 40-80 % G-residues or 40-75 % G-residues (monomer units).

In another embodiment, the biocompatible hydrogel particles are made of alginate with a G:M ratio ≥ 0.4, such as a G:M ratio ≥ 0.5, a G:M ratio ≥ 0.7, a G:M ratio ≥ 0.9, a G:M ratio ≥ 1, a G:M ratio ≥ 1.2, a G:M ratio ≥ 1.4 or a G:M ratio ≥ 1.5.

In one embodiment, the biocompatible hydrogel particles are made of a mixture of at least two different alginates, wherein each of the alginates independently has a G:M ratio ≥ 0.4, such as a G:M ratio ≥ 0.5, a G:M ratio ≥ 0.7, a G:M ratio ≥ 0.9, a G:M ratio ≥ 1, a G:M ratio ≥ 1.2, a G:M ratio ≥ 1.4 or a G:M ratio ≥ 1.5.

In one embodiment, the biocompatible hydrogel particles are made of alginate with a molecular weight in the range of 10-300 kDa, such as 10-250 kDa, 10-200 kDa, 10-150 kDa, 10-100 kDa, 50-300 kDa, 50-250 kDa, 50-200 kDa, 50-150 kDa or 50-100 kDa.

In one embodiment, the biocompatible hydrogel particles are made of a mixture of at least two different alginates, wherein each of the alginates independently has a molecular weight in the range of 10-300 kDa, such as 10-250 kDa, 10-200 kDa, 10-150 kDa, 10-100 kDa, 50-300 kDa, 50-250 kDa, 50-200 kDa, 50-150 kDa or 50-100 kDa.

**The biocompatible hydrogel particles may easily be removed** from the vascularized extracellular matrix in case the intended use requires a product without hydrogel particles. Removal of the particles may be done by subjecting the vascularized extracellular matrix to treatment suitable for dissolving and/or degrading the biocompatible hydrogel particles. The treatment to be used depends on the composition of the biocompatible hydrogel particles and are well known to the skilled person.

In one embodiment, the biocompatible hydrogel particles are self-degradable or self-dissolvable. The terms "self-degradable" and "self-dissolvable" means that the biocompatible hydrogel particle will degrade or dissolve by itself. Thus, there is no need to add any agents in order for the degradation or dissolution to take place. The time needed for the degradation or dissolution to be completed will typically decrease with increasing temperatures.

The **cell-particle mixture is cultured** to obtain the vascularized extracellular matrix, preferably cultured in a mold of a desired shape and size. The vascularized extracellular matrix formed will typically take the shape of the mold used.

Conditions and culture media suitable for culturing the cell-particle mixture is well known to the skilled person. However, various growth conditions can be selected to enhance the process of extracellular matrix production, and/or stimulate the development of desirable mechanical, physical, or biochemical properties. Such growth conditions may include the use of growth media supplemented with amino acids such as proline, alanine, glycine and/or ascorbic acid to enhance extracellular matrix protein production. Additionally, the growth media may be supplemented with growth factors such as FGF2 (fibroblast growth factor) and/or any other growth factor that may be important for control of extracellular matrix production and degradation and the interaction between cells and extracellular matrix.

It has previously been discussed that the first population of cells, in particular MSCs, proliferate fast while the growth of the second population of cells, in particular HUVECs, is very limited due to the need to self-organize into capillaries. The outgrowth of the first population of cells may limit nutrient access to the second population of cells over time, leading the second population of cells to a premature death. The risk of a premature death for the second population of cells may be reduced by having an appropriate ratio between 'the number of cells in the first population of cells' and 'the number of cells in the second population of cells'.

However, not only the ratio between the two cell types, but also the level of nutrients in the cell media over time may affect the efficiency of the method and the quality of the product. Hence, in one embodiment, the cell-particle mixture in step b) is cultured in a cell media for a predetermined period of time; and the cell media is partly or completely replaced by fresh cell media at an interval sufficient to ensure that the level of glucose in the cell media is above 1 mmol/l during the predetermined period of time; wherein the predetermined period of time preferably is more than 7 days, such as more than 15 days.

The cell-particle mixture may be cultured in the mold to produce vascularized extracellular matrix at least until the vascularized extracellular matrix can be removed from the mold and still maintain its shape, i.e. that the vascularized extracellular matrix is self-supporting.

The cell-particle mixture may be cultured in a **mold** to obtain the vascularized extracellular matrix. The vascularized extracellular matrix formed may take the shape of the mold used.

The mold may be made of a bioinert material and provide exterior support for the vascularized extracellular matrix until it is self-supporting. Hence, in one embodiment, the mold has one or more of the following properties: (1) allow control of geometry; (2) prevent cell attachment and/or infiltration; and (3) allow exchange of nutrients and gases between exterior and interior of the mold. In one embodiment, the mold is completely or partly submerged in cell culture media. The mold may have the shape of an annular well. Preferably, the mold allows manufacturing vascularized extracellular matrix of any desired shape and/or complex geometry.

The vascularized extracellular matrix may be repopulated by cells. The vascularized extracellular matrix may be suitable for being implanted into a subject, such as a human subject.

A **second aspect** relates to a vascularized extracellular matrix obtainable or obtained by the method according to the first aspect.

A **third aspect** relates to a vascularized extracellular matrix comprising:
- extracellular matrix, preferably human extracellular matrix;
- capillaries embedded within the extracellular matrix;
- a first population of cells embedded within the extracellular matrix;
- a second population of cells embedded within the extracellular matrix; and
- biocompatible hydrogel particles embedded within the extracellular matrix;
wherein the first population of cells produce and secrete the extracellular matrix; and the second population of cells are endothelial cells that form the capillaries embedded within the extracellular matrix.

The second- and third aspects relate to supportive matrices which may serve as an intermediate in the production of a new functional tissue. The supportive matrices may be repopulated and cultured *in-vitro.* The *in-vitro* prepared tissue may be implanted into a human subject or used for *in-vitro* testing purposes. Hence, a **fourth aspect** relates to *in-vitro* use of the vascularized extracellular matrix according to the second- or third aspect for growing bioartificial tissue, preferably as a supportive matrix for growing bioartificial tissue. Optionally, the supportive matrices may be implanted into a subject, such as a human subject, where it is repopulated by host cells *in-vivo.*

**A fifth aspect** relates to the vascularized extracellular matrix according to the second- or third aspect for use in treatment of skin wounds by enhancing angiogenesis and vascularization.

**A sixth aspect** relates to the vascularized extracellular matrix according to the second- or third aspect for use in a surgical procedure for replacing and/or repairing blood vessels in a subject, preferably a human subject.

A **seventh aspect** relates to a composition comprising a first population of cells, a second population of cells, and biocompatible hydrogel particles; wherein the first population of cells can produce and secrete extracellular matrix, the second population of cells being endothelial cells that can form capillaries within the extracellular matrix, and the number of cells in the first population of cells : number of cells in the second population of cells is in the range 1 : 10 to 10 : 1, more preferably in the range 1 : 5 to 5 : 1 and most preferably in the range 1 : 3 to 3 : 1. This composition is the cell-particle mixture referred to in step a) of the first aspect.

Having generally described this invention, a further understanding can be obtained by reference to certain specific examples, which are provided herein for purposes of illustration only, and are not intended to be limiting unless otherwise specified.

### Examples

### Example 1: Manufacturing of vascularized extracellular matrix

### 1.1 Cell culturing

Adipose-derived Mesenchymal Stromal Cells (MSCs) were acquired from Lonza (cat.n. PT-5006) (AT0). MSCs were cultured in αMEM (Gibco, cat.n. 32561), supplemented with 10% FBS, 1% Pen/Strep (Sigma cat.n. P4333-100ML), EGF (10 ng/ml), Ascorbic Acid (75 ug/ml), Hydrocortisone (0.8 ng/ml), insulin (10 ug/ml) (CCM) and used at passage number 6.

HUVECs were acquired from Lonza, (cat.n. CC-2517). HUVECs were cultured in Lonza proprietary media formulation composed of EBM-2 media (cat.n. CC-3156) supplemented with SingleQuot MV kit (cat.n. CC-4147), denominated EGM-2 and used at passage number 9.

### 1.2 Preparation of biocompatible hydrogel beads

Alginate microbeads were produced using a 2-stream flow focusing droplet chip (Wunderlichips, Switzerland) with a 50x50 µm droplet forming nozzle. The continuous phase consisted of Novec 7500 fluorinated oil (3M, US) containing 1% Pico-Surf (Sphere Fluidics, UK) as a surfactant.

Two dispersed phases were used: (1) 1.0% (wt) total alginate consisting of 0.5% NOVATACH^{™} VLVG 4GRGDSP (4GRGDSP-coupled high G low MW alginate, G/M Ratio ≥ 1.5, nominal viscosity 10-80 mPa.s, 69% guluronate monomer units, 33 kDa) and 0.5% PRONOVA^{™} UP LVG (20-200 mPa.s, 70% guluronate monomer units, 108 kDa), containing 80 mM CaEDTA and 20 mM HEPES at pH 7.0 and (2) 1.0% (wt) total alginate consisting of 0.5% NOVATACH^{™} VLVG 4GRGDSP (4GRGDSP-coupled high G low MW alginate, G/M Ratio ≥ 1.5, nominal viscosity 10-80 mPa.s, 69% guluronate monomer units, 33 kDa) and 0.5% PRONOVA^{™} UP LVG (20-200 mPa.s, 70% guluronate monomer units, 108 kDa) containing 80 mM ZnEDDA and 20 mM HEPES at pH 7.0.

The two aqueous phases meet in a co-flow region in the microfluidic channels prior to droplet break-up. The flow rates were set to 1000 µL h⁻¹ for the continuous phase and 200 µL h⁻¹ for both aqueous phases by controlled injection using BD plastic syringes with PE plastic tubing (Scientific Commodities Inc.) mounted on syringe pumps (Harvard Apparatus, PHD ULTRA).

The microbeads were collected, and surfactant removed by adding perfluoro octanol (PFO, TCI Europe) at a 1:1 ratio to produced bead volume. Beads were centrifuged at 1000 relative centrifugal force (rcf) for 1 min, non-aqueous phase was removed, then rinsed with complete growth medium at a 2:1 volume to collected beads and washed again. Any remaining non-aqueous residue was removed by pipette. Final processed beads were stored at 4°C for at least 12 hours in complete growth media to swell to equilibrium size.

In order to measure bead size, an aliquot was taken, diluted 1:10 in complete growth medium, and applied to a glass slide. A transmitted light image was taken using an inverted optical light microscope and 4x objective. Beads were detected in the image using an algorithm based on circular Hough transform. The diameter of the detected beads was then quantified from the image using the established pixel/micron ratio of the calibrated microscope, with a resulting size of 59.2 +/- 1.4 µm (SD). The bead concentration was counted manually using a hemocytometer.

### 1.3 Preparation of vascularized extracellular matrix

MSCs (example 1.1) and HUVECs (example 1.1) are grown to near-confluence level (90-100%) in cell stack 5 (Corning, cat.n. 3313) and cell stack 2 (Corning, cat.n. 3269), respectively. Cells are then detached using TrypLE (Gibco, cat.n. 12605028) for MSCs and trypsin-EDTA 0.25% for HUVECs (Gibco, cat.n. 25200-056). Cells are subsequently resuspended in their own media and an aliquot is counted using Countess (Life Technologies, cat.n. A25750rev01).

75 × 10⁶ MSCs (example 1.1) is mixed with 25 × 10⁶ HUVECs (example 1.1) and 41.4 × 10⁶ hydrogel beads (example 1.2). The mixture is then incubated at 37° C for 2 hours with gentle agitation every 30 minutes. Subsequently, the mixture is centrifuged at 250 xg for 8 minutes without brakes. The supernatant is removed as much as possible without disturbing the pellet to obtain a highly concentrated mixture (containing a total of 15-16 ×10⁶ cells/ml). The highly concentrated preparation is then gently mixed with a P1000 pipette and subsequently aspirated into a syringe before it is injected into a mold (the mold being the tissue cassette disclosed in EP24214592 having the following dimensions H 3 cm x L 2 cm x D 1.3 mm and a total capacity of 1 ml). The mold is placed inside a jar (Nalgene, cat.n. 2118-0008) containing 180 ml of media (composed of a 1:1 mixture of CCM and EGM-2). The mold is incubated at 37° C with a magnetic stirrer for 21 days with 25 % v/v media refresh every 3 days (i.e. 25 % v/v of the media in the jar is replaced by fresh media every third day throughout the 3 weeks incubation period). The alginate beads were then removed. After 21 days in culture, the vascularized extracellular matrix was removed from the mold (demolded).

### 1.4 Histology and fluorescent staining

The demolded vascularized extracellular matrix obtained in example 1.3 was washed briefly with PBS and then fixed in 10% NBF (Sigma, cat.n. HT501128-4L), equivalent to 4% PFA, for 30 minutes at room temperature. Vascularized extracellular matrices are then washed 3 times in PBS and then a small piece of the sample is excised and sent for paraffin-embedding, sectioning and staining by Hematoxylin & Eosin (figure 2a and 2b) or immunofluorescence on sections using antiCD31 as marker for mature endothelial cells (figure 3) and Smooth Muscle Actin (SMA) as a marker for differentiating MSCs (figure 4a and 4b).

For confocal imaging (figures 1a-c), a tissue piece was blocked in PBS-BSA 2% for 1 hour at RT and then ULEX-AF488 (Invitrogen, cat.n. L32476) is diluted 1:500 in PBS-BSA 1% and incubated with the tissue for 1 hour at RT. Tissue is then washed 3 times with PBS and then Hoechst 33420 is added (diluted 1: 10000 in PBS) to stain nuclei.

## Claims

1. An *in-vitro* method of manufacturing vascularized extracellular matrix, the method comprising the following steps:
a) bringing a first population of cells and a second population of cells together with biocompatible hydrogel particles to obtain a cell-particle mixture;
b) culturing the cell-particle mixture to obtain the vascularized extracellular matrix;
wherein
- the first population of cells produce and secrete extracellular matrix;
- the vascularized extracellular matrix comprises capillaries embedded within the extracellular matrix;
- the second population of cells being endothelial cells that form the capillaries embedded within the extracellular matrix; and
- the number of cells in the first population of cells : number of cells in the second population of cells, in step a), is in the range 1 : 10 to 10 : 1, more preferably in the range 1 : 5 to 5 : 1 and most preferably in the range 1 : 3 to 3 : 1.

2. The method according to claim 1, wherein
- the cells in the first population of cells and/or the cells in the second population of cells are anchorage-dependent cells; and
- the biocompatible hydrogel particles support attachment of the first population of cells and/or the second population of cells.

3. The method according to any one of the preceding claims, wherein the method further comprises step c), where the vascularized extracellular matrix obtained in step b) is subjected to treatment for dissolving and/or degrading the biocompatible hydrogel particles.

4. The method according to any one of the preceding claims, wherein
- the cell-particle mixture in step b) is cultured in a mold to obtain the vascularized extracellular matrix; and
- the mold is designed to allow the exchange of nutrients and gases between exterior and interior of the mold, and the mold is completely or partly submerged in cell culture media during cultivation.

5. The method according to any one of the preceding claims, wherein the biocompatible hydrogel particles are biocompatible hydrogel beads, biocompatible hydrogel fibers or any mixture thereof; more preferably biocompatible alginate beads, biocompatible alginate fibers or any mixture thereof.

6. The method according to any one of the preceding claims, wherein the biocompatible hydrogel particles are biocompatible hydrogel beads, more preferably biocompatible alginate beads.

7. A vascularized extracellular matrix obtainable or obtained by the method according to any one of claims 1-6.

8. A vascularized extracellular matrix comprising:
- extracellular matrix, preferably human extracellular matrix;
- capillaries embedded within the extracellular matrix;
- a first population of cells embedded within the extracellular matrix;
- a second population of cells embedded within the extracellular matrix; and
- biocompatible hydrogel particles embedded within the extracellular matrix;
wherein
the first population of cells produce and secrete the extracellular matrix; and the second population of cells being endothelial cells that form the capillaries embedded within the extracellular matrix.

9. The vascularized extracellular matrix according to claim 8, wherein
- the first population of cells and/or the second population of cells are anchorage-dependent cells; and
- the biocompatible hydrogel particles support attachment of the first population of cells and/or the second population of cells.

10. The vascularized extracellular matrix according to any one of claims 8-9, wherein the biocompatible hydrogel particles are biocompatible hydrogel beads, biocompatible hydrogel fibers or any mixture thereof, more preferably biocompatible alginate beads, biocompatible alginate fibers or any mixture thereof.

11. The vascularized extracellular matrix according to any one of claims 8-10, wherein the biocompatible hydrogel particles are biocompatible hydrogel beads, more preferably biocompatible alginate beads.

12. The method according to any one of claims 1-6 or the vascularized extracellular matrix according to any one of claims 7-11, wherein the vascularized extracellular matrix is suitable for being implanted into a subject, preferably a human subject.

13. *In-vitro* use of the vascularized extracellular matrix according to any one of claims 7-12, as a supportive matrix for growing bioartificial tissue.

14. The vascularized extracellular matrix according to any one of claims 7-12, for use in treatment of skin wounds.

15. The vascularized extracellular matrix according to any one of claims 7-12, for use in a surgical procedure for replacing and/or repairing blood vessels in a subject, preferably a human subject.

16. A composition comprising:
- a first population of cells;
- a second population of cells; and
- biocompatible hydrogel particles;
wherein
- the first population of cells can produce and secrete extracellular matrix;
- the second population of cells being endothelial cells that can form capillaries within the extracellular matrix; and
- the number of cells in the first population of cells : number of cells in the second population of cells is in the range 1 : 10 to 10 : 1, more preferably in the range 1 : 5 to 5 : 1 and most preferably in the range 1 : 3 to 3 : 1.
